# EUROPEAN PATENT SPECIFICATION

(11) **EP 1 841 527 B1**
(45) Date of publication and mention of the grant of the patent: **30.09.2009**
(21) Application number: 05854430.5
(22) Date of filing: 16.12.2005
(51) Int. Cl.: B01J 20/22

(54) **CHROMATOGRAPHIC MEDIA**
CHROMATOGRAPHISCHES MEDIUM
SUPPORTS CHROMATOGRAPHIQUES

(30) Priority: 25.01.2005 US 646780 P
(43) Date of publication of application: 10.10.2007
(73) Proprietor: Mallinckrodt Baker, Inc., Phillipsburg, New Jersey 08865 (US)
(72) Inventor: DEORKAR, Nandu, Cedar Knolls, New Jersey 07927 (US); BUSS, Robert, C., Allentown, Pennsylvania 18103-4220 (US); MLADOSICH, Joseph, M., Nazareth, Pennsylvania 18064 (US); BOUIS, Paul, A., Bethlehem, Pennsylvania 18020 (US)
(74) Representative: Cockerton, Bruce Roger
(86) International application number: PCT/US2005/045710
(87) International publication number: WO 2006/081002

(56) References cited:
- EP-A- 1 226 870
- DE-A1- 4 113 602
- DE-A1- 19 740 770
- ROUNDS ET AL: "Synthesis of a non-porous, polystyrene-based strong anion-exchange packing material and its application to fast high-performance liquid chromatography of proteins" JOURNAL OF CHROMATOGRAPHY, vol. 443, 1988, pages 73-83, XP002394091 Amsterdam
- DENIZLI A ET AL: "Mercury removal from synthetic solutions using poly(2-hydroxyethylmethacrylate) gel beads modified with poly(ethyleneimine)" REACTIVE & FUNCTIONAL POLYMERS, ELSEVIER SCIENCE PUBLISHERS BV, NL, vol. 55, no. 2, April 2003 (2003-04), pages 121-130, XP004421374 ISSN: 1381-5148

## Description

### Field of the Invention

The invention relates to the preparation and use of novel chromatographic media, preferably mixed mode polymeric chromatographic media for separation and purification of various biomolecules such as peptides, proteins and antibodies. More particularly, the present invention discloses novel chromatographic media, preferably mixed anion exchangers, mixed anion-cation exchangers and hydrophobic exchangers. The chromatographic media is prepared by modification of polymers with polyethyleneimine and functionalization thereof. It has been unexpectedly discovered that these mixed mode polymeric media offer enhanced separation capability and protein binding capacity.

### Background of the Invention

The analysis of protein mixtures by ion exchange chromatography media is well documented, such as by, Pete Gagnon, " Purification Tools for Monoclonal Antibodies", Validated Biosystems, Inc., (1996). More recently, the development of protein-based drugs and vaccines has increased the need for larger scale purification of protein mixtures. It is highly desirable that ion exchange chromatographic media be able to be utilized in this area.

One way of preparing such chromatography media materials, particularly anion exchangers containing primary and secondary amine functionality, is by coating the internal surface of porous silica materials with polyethyleneimine (PEI). For example, coating of the internal surface of silica particle with polyethyleneimine followed by immobilization through crosslinking has been disclosed in Alpert and Regnier, J. Chromatogr. 185, 375 - 392 (1979), and the use of materials prepared in that fashion for the chromatographic separation of synthetic oligonucleotides has been described in Lawson et al., Anal. Biochem. 133, 85-93 (1983).

Similarly, the preparation of PEI coated porous silica particles or controlled pore glass particles obtained by covalent binding of polyethyleneiminopropyl-trimethoxy silane has been disclosed in US 4,540,486 of JT Baker Chemical Co. The same patent discloses the formation of chromatographic resins having mixed weak base/weak acid functionality by reaction of the PEI coated silica with cyclic carboxylic anhydrides. It has been shown that these materials, when used in a chromatographic column, can separate mixtures of cytochrome C, alfa1-acid glycoprotein, ovalbumin and beta-lactoglobulin (weak base media) or ovalbumin, cytochrome C, hemoglobin and lysozyme (mixed weak base/weak acid media). The efficacy of the PEI derivatized porous silica, or its carboxylated version in purifying immunoglobulin G has also been demonstrated in US 4,606,825 of JT Baker Chemical Co.

The acylated form of the PEI coated silica described above can be further converted into a mixed weak acid/strong acid functionality media by the introduction of sulfonic groups, as disclosed in US 4,721,573 of JT Baker Chemical Co. A column packed with this sulfonated media allows the separation of a mixture of cytochrome C, hemoglobin, lysozyme and ovalbumin, as well as the separation of the protein components of hybridoma cell culture medium.

Silica particles to which PEI has been covalently bound can also be converted to hydrophobic chromatographic media by reaction with monoacyl chlorides or linear carboxylic anhydrides where the acyl group can be a linear hydrocarbon chain, a phenyl group or a substituted phenyl group. A silica based weak base/reverse phase chromatographic media prepared in that fashion using butyryl chloride separates cytochrome C, myoglobin, lysozyme ovalbumin and alpha-chympotrypsinogen, as disclosed in US 4,551,245 of JT Baker Chemical Co.

Agarose beads bearing ion exchange groups at the end of a polyvinyl alcohol (PVA) arm has been disclosed In WO 98/58732 to Amersham. However, unlike in the present invention, the PVA spacer arms do not contribute any ion exchange capacity to the product, nor does it in Itself provide one of the functionalities in a mixed mode product.

One of the major disadvantages of silica based chromatographic packings Is their lack of stability at high pH. This is particularly the case for application involving the purification of drugs, since treatment of the equipment with 1 N sodium hydroxide is a common sterilization practico.

### SUMMARY OF THE INVENTION

The present invention provides chromatographic media that is able to minimize or avoid this lack of stability at high pH. The chromatographic media of the invention is set out in claim 1. The invention also provides mixed mode chromatographic media. It has been discovered that one possible way to avoid this instability problem is to use chromatographic packings based on a polymeric support derivatized on the surface of the polymer (i.e. not cross-linked) with PEI, which surface derivatized media can in accordance with the invention, be further functionalized by reaction of a functionalization reagent with terminal amino groups of the polyethyleneimine on the surface of the polymeric resin as set out in claim 1. In accordance with this invention there preferably can be provided mixed mode media, such as for example, media with mixed primary, secondary and tertiary amine exchange sites, media with both weak anion and weak cation exchange sites, media with weak anion, weak cation and strong cation exchange sites, media with weak anion and hydrophobic (reverse phase) exchange sites, and media with weak anion and strong anion. Furthermore, it Is unexpectedly discovered that such polyethyleneimine derivatized polymeric and functionalized derivatives thereof-based chromatographic media provide different and unique separation characteristics. In one aspect of the present invention there is provided polymeric chromatographic media for bioseparations. This invention differs from current polymeric chromatographic media by method of preparation and characteristics of the media as these current chromatographic media are simple Ion exchangers. Also, the media of the present invention differs from current silica based media in terms of method of preparation, composition, use and performance. Surprisingly, polymeric mixed mode media prepared according to this invention have enhanced separation and binding capacities.

### Brief Description of the Drawings

The present invention is illustrated by, but not limited by, the accompanying drawings in which:
Figure 1 (illustrative) is a graph of the elution profile, as recorded by a UV detector, of the separation of proteins using media prepared according to preparative Example 1 and PEI silica media according to the procedure Example 34;
Figure 2 is a graph of the elution profile, as recorded by a UV detector, of the separation of proteins using media prepared according to example 9 and mixed mode silica media according to the procedure Example 35;
Figures 3 a and 3 b are graphs of the elution profile, as recorded by a UV detector, of the separation of proteins using media prepared according to Example 19 and silica media according to the procedure Example 37;
Figure 4 is a graph of the elution profile, as recorded by a UV detector, of the separation of proteins using media prepared according to Example 20 according to the procedure of Example 38;
Figure 5 is a graph of the solution profile, as recorded by a UV detector, of the separation of proteins using media prepared according to Example 22 according to the procedure In Example 39;
Figure 6 is a graph of the elution profile, as recorded by a UV detector, of the separation of proteins using media prepared according to Example 23 according to the procedure in Example 40;
Figure 7 is a graph of the acid stability of media prepared according to Example 7 as determined according to the procedure in Example 41; and
Figure 8 is a graph of the base stability of media prepared according to Example 7 as determined according to the procedure of Example 42.

### Detailed Description of the Invention

This invention concerns chromatographic media, and the preparation and use of novel polymeric chromatographic media and preferably mixed mode polymeric chromatographic media. In accordance with the present invention, polymeric media is prepared using polymeric particles derivatized with polyethyleneimine and functionalized with appropriate reactants, as set out in claim 1.

Polymeric materials used for the chromatographic separation of proteins will preferably have certain properties, such as,
1) the pore sizes is sufficiently large to allow rapid diffusion of molecules as large as protein in and out of the resin particles;
2) interactions between the proteins and the non-functionalized polymer are to be weak to avoid "non specific interaction" and allow recovery of the desired protein in high yields;
3) the resin particles are to be rigid to avoid compassion and loss of flow rate under the pressure encountered in chromatographic operations; and
4) the resin should be chemically stable under all conditions encountered in the separation process.

The polymeric resin particles to be surface derivatized with polyethyleneimine can be any suitable polymeric resin particles capable of being derivatized with polyethyleneimine and, when functionalized as set out in claim 1, being useful as chromatographic separation media. Examples of polymeric resin particle suitable for derivatization with polyethyleneimine in accordance with this invention Include, but are not limited to cellulose, agarose, epoxidized or halogenated polystyrenes, epoxidized or halogenated polyacrylates or polymethacrylates, and epoxidized or halogenated polydivinylbenzenes. For examples, porous poly(meth)acrylates, highly cross-linked resins based on (meth)acrylic monomers bearing multiple polymerizable double bonds, such as ethylene glycol dimethacrylate (US 4,110,347 to Showa Denko, pentaerythritol trimethacrylate (US 4,256,842 to Toyo Soda), trimethylol propane trimethacrylate (US 4,582,860 to Rohm and Haas) or glycerol dimethacrylate (US 2,254,634 to Mitsubishi) prepared in the presence of a pore forming agent, have been shown to provide materials with the desired properties. Furthermore, the addition of functional monomers to the polymerization mixture affords final product with functional groups to which other molecules can be bound through the formation of covalent bonds. Example of such monomers are glycerol methacrylate (-OH groups) (US 2,254,634 to Mitsubishi, 1993), dimethylaminoethyl methacrylate (tertiary amines) or glycidyl methacrylate (US 4,118,347 to Showa Donko, US 4,256,842 to Toyo Soda, US 4,582,860 to Rohm and Haas). Functionalization reagents for reaction with the PEI surface-derivatized polymeric resin particles are acid anhydrides such as cyclic carboxylic anhydrides such as glutaric and succinic anhydrides, unsaturated carboxylic anhydrides such as maleic anhydride, sulfonation agents such as bisulfites such as sodium meta-bisulfite, alkyl chlorides or anhydrides such as butryl chloride and acetic or butyric anhydride,and alkyl chlorides containing quaternary ammonium functionality such as (3-chloro-2-hydroxypropyl)trimethylammonium chloride, and mixtures of these functionalization reagents.

In one embodiment of this invention, an anion exchanger with mixed primary and secondary and tertiary amine sites is prepared by reacting polyethyleneimine with a polymeric support bearing epoxy or halo groups such as chloro, bromo, iodo groups. Such polymeric; support can be any suitable synthetic polymer or natural polymer resin such as poly(meth)acrylate, cellulose, polystyrene divinylbenzene, and agarose. Examples of such commercially available resins are Tosoh Biosciences Toyopearl AF-epoxy 650M, epoxy activated Sepharose 6B. These materials can be reacted with one of the terminal amino group of polyethyleneimine of various molecular weights through the formation of chemically stable alpha-hydroxy amino groups. Some of the properties of materials prepared in that manner, according to following preparative examples 1 through 5, are summarized in table

In a second embodiment, mixed mode media with weak anion and weak cation exchange sites are prepared by reacting the PEI functionalized polymeric beads with cyclic carboxylic acid anhydrides, such as glutaric or succinic anhydride in the following examples 6 to 10.

In a third embodiment, mixed modo media having weak anion, weak cation and strong cation sites were prepared by reacting the PEI derivatized polymer with an unsaturated carboxylic acid anhydride followed by sulfonation, as described in examples 11 through 13.

In a fourth embodiment, mixed mode media having weak anion and hydrophobic (reverse phase) sites were prepared by reacting the PEI derivatized polymer with alkyl chloride (examples 14 through 18) or monobasic acids anhydrides Examples 19 through 24). Reactions with butyryl chloride were performed for 2 hr. at room temperature using either toluene or dioxane as the solvent. Triethylamine was used to scavenge the hydrochloric acid produced as a by-product of the reaction.

In a fifth embodiment, mixed mode media are obtained by reaction of the PEI coated resin with butyric anhydride conducted in 1-methoxy-2-propanol for 3 hr. at 60 °C. as set forth in following Examples 20-22.

Mixed mode weak base/reverse phase resins where the hydrophobic moiety is a methyl group or a combination of methyl and butyl groups can be prepared by using acetic anhydride or sequential addition of butyric and acetic anhydride, as shown in Example 24 (acetic anhydride) or in Examples 20 and 23 (mixed anhydrides) respectively. As such, it is possible to vary and select proper hydrophobiciety by using one to a combination of reagents.

In a sixth embodiment, mixed mode media having weak anion, and strong anion (quaternary ammonium) sites were prepared by reacting the PEI derivatized polymer with alkyl chlorides containing terminal quaternary ammonium functionality. More particularly, the PEI derivatized polymer is reacted with (3-chloro-2-hydroxypropyl) trimethylammonium chloride in aqueous sodium hydroxide at 70-80 °C as shown In following Examples 25 to 30.

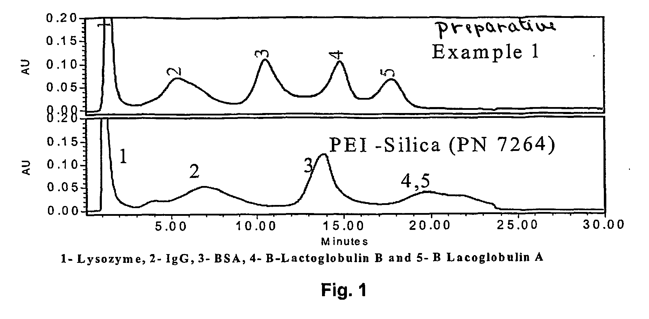

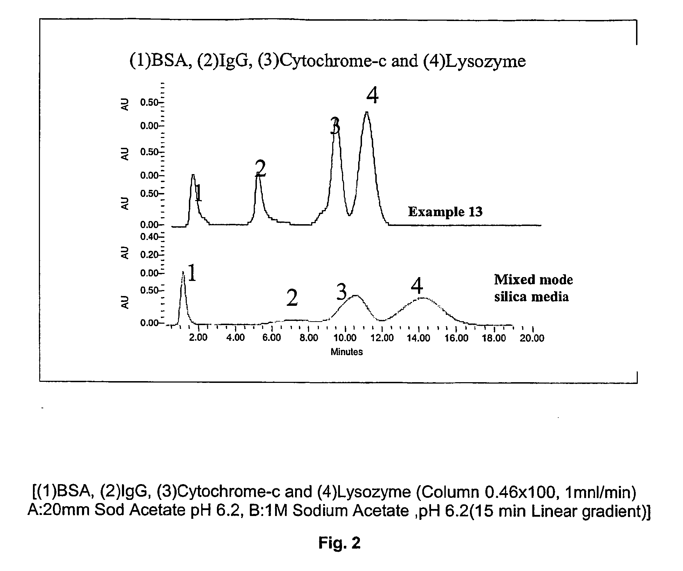

In accordance to the present invention, it has been unexpectedly discovered that these polymeric mixed mode chromatographic media offer enhanced separation capability as well as high capacity and stability. For example, the polymeric mixed mode anion exchange media can separate lysozyme, immunoglobulin G, bovine serum albumin, beta- lactoglohulin A and beta-lactoglobulin B while PEI-silica based media cannot separate all of these proteins as shown in Figure 1. The mixed mode cation exchange media can separate BSA, IcG, Cytochrome-C and Lysozyme efficiently, as shown in Figure 2, while the mixed mode silica media cannot separate all these proteins.

EXAMPLES

### Example 1 (preparative)

12 g epoxy bearing methacrylate polymer with average particle size of 35 micron diameter and 250 ml dioxane are placed in a 1 L round bottom flask equipped with a funnel, agitator, reflux condenser and positive nitrogen pressure inlet and stirred for 30 min to allow the resin to swell. 20 g polyethyleneimine (PEI, average molecular weight 600 Daltons) is added and the funnel rinsed with an additional 150 ml dioxane. The stirred mixture is refluxed overnight. After allowing the mixture to cool, the mixture is transferred to a filter flask, drained, washed once with dioxane, three times with methanol and dried under vacuum at 60°C. Elemental analysis: 57.8% C, 7.4% H and 5.6 % N.

### Example 2 (preparative)

5 g epoxy bearing methacryate polymer and 150 ml of dioxane are placed in a 500 ml round bottom flask equipped with an agitator and reflux condenser. 30 g of PEI (average molecular weight 1200 Daltons) are added and the mixture is refluxed overnight. After allowing to cool, the mixture is transferred to a filter flask, drained, washed twice with dioxane, three times with methanol and dried. Elemental analysis: 55.7% C, 7.8 % H, 5.5 % N

### Example 3 (preparative)

5 g epoxy bearing methacrylate polymer and 150 ml of a 50/50 mixture of dioxane and water are placed in a 500 ml round bottom flask equipped with an agitator and reflux. 30 g of PEI (average molecular weight 1200 Daltons) are added and the mixture is refluxed overnight (89 °C). After allowing to cool, the mixture is transferred to a filter flask, drained, washed twice with dioxane, three times with methanol and dried. Elemental analysis indicates that the final product contains: 67.5% C, 7.4 % H, 2.5 % N

### Example 4 (preparative)

5 g epoxy bearing methacrylate polymer and 100 ml of a 30 wt % solution of 10,000 daltons average molecular weight PEI in dioxane are placed in a 250 ml round bottom flask equipped with an agitator, nitrogen inlet and reflux condenser and the stirred mixture is refluxed overnight. After allowing to cool, the mixture is transferred to a filter flask, drained, washed three times with 60 °C water, three times with methanol and dried overnight in a vacuum oven at 60 °C. Elemental analysis indicates that the final product contains: 56.5% C, 7.8 % H, 6.4 % N

### Example 5 (preparative)

5 g epoxy bearing methacrylic resin and 100 ml of a 30 wt % solution of 10,000 daltons average molecular weight PEI in water are placed in a 250 ml round bottom flask equipped with an agitator and reflux condenser and the stirred mixture is refluxed for 17 1/2 hr. After allowing to cool, the mixture is transferred to a filter flask, drained, washed three times with water, three times with methanol and dried overnight in a vacuum oven at 60 °C. Elemental analysis indicates that the final product contains 56.9% G, 7.5% H and 3.2% N.

As the results in Table 1 indicate, the presence of water in the system has a detrimental effect on nitrogen incorporation, while similar nitrogen contents are obtained when PEI of various molecular weight are reacted with the resin In dry dioxane (albelt at a lower PEI to polymer ratio than in example 1).

**Table 1. Effect of solvent and PEI molecular weight on nitrogen incorporation**

| | | | |
|---|---|---|---|
| Solvent | 600 Daltons PEI | 1,200 Daltons PEI | 10,000 Daltons PEI |
| Dioxane | Example 1 PEI/polymer = 1.66 5.6%N | Example 2 PEI/polymer = 6 5.5% N | Example 4 PEI/polymor = 6 6.4 % N |
| Water/dioxane (1:1) | N/A | Example PEI/polymer = 6 2.5% N | N/A |
| Water | N/A | N/A | Example 5 PEI/gel = 6 3.2%N |

As the results in Table 1 further indicate, the reactions performed in pure dioxane, as opposed to dioxane/water mixture or pure water, give a higher degree of functionalization, as indicated by the nitrogen content of the final product. When pure dioxane is used as the solvent (first row of Table 1), there does not appear to be a significantly higher level of nitrogen introduction as the molecular weight of the PEI is increased from 600 to 10,000 Daltons.

### Example 6:

30 g PEI polymer prepared as in example 1 is washed with twice its volume of 100 % ethanol and twice with its volume of 1-methoxy-2-propanol to remove any residual moisture. The polymer is then slurred in 450 ml 1-methoxy-2-propanol, transferred to a flask equipped with overhead agitator, reflux condenser and positive nitrogen pressure inlet, and heated to 60 °C. After adding a solution of 19.43 g of 95% glutaric anhydride (1 eq, based on nitrogen content of the PEI resin) the temperature is maintained at 60 +/-2 °C for 2 1/2 hr. The reaction mixture is then transferred to a filter flask, drained, and the residual solid washed once with 100ml of 1-methoxy-2-propanol, twice with 100ml methanol and twice with 100ml storage solution (ethanol:water 20:80 v/v, or 100mM sodium acetate pH 4.5 with 2% benzyl alcohol). The product was stored in the respective storage solution for characterization and for chromatographic separation of proteins. Elemental analysis indicates that the final product contains: %C=55.1, %N=4.6.

### Example 7

73.7 g PEI coated polymer prepared as in example 1, 225 ml 1-methoxy-2-propanol and 14.4 g succinic anhydride are heated to 60 +/-2 °C and maintained at that temperature for 2 1/2 hr. The reaction mixture is then transferred to a filter flask, drained, and the residual solid washed once with 100ml of 1-methoxy-2-propanol, twice with 100 ml methanol and twice with 100ml of storage buffer solution as described in example 5. Elemental analysis indicates that the final product contains: 53.08 %C, 7.13% H and 4.86 % N

### Example 8

The reaction was conducted as in Example 7 except the succinic anhydride charge is 17.34 g. Elemental analysis indicates that the final product contains: 53.18 %C, 7.97% H and 5.18 % N

### Example 9

11 g PEI coated polymer prepared as in Example 1 is washed with twice its volume of 100 % ethanol and twice with its volume of dioxane to remove any residual moisture. The resin is then slurred in about 200 ml dioxane, transferred to a three flask equipped with a nitrogen inlet tube and heated to 50 - 60 °C. After one hour, 7 g glutaric anhydride are added and the temperature maintained at 60 +/-2 °C for 2 1/2 hr. The reaction mixture is then transferred to a filter flask, drained, and the residual solid washed three times with dioxane, three times with methanol and dried under vacuum. Elemental analysis indicates that the final product contains: 56.64 %C, 7.54 % H and 4.36% N.

### Example 10

The reaction was conducted as in Example 9 except 10 g PEI polymer prepared according to Example 1 and 6.6 g succinic anhydride are used. Elemental analysis indicates that the final product contains: 54.46 %C, 7.96 %H and 4.6%N.

**Table 2. Preparation of media with weak anion and weak cation exchange sites.**

| Example | Solvent | ml solvent/ g resin | Anhydride | meq. anhydride/ g resin | Temp °C | Time Hrs | %N |
|---|---|---|---|---|---|---|---|
| | | | | | | | |
| 6 | 1-M-2-P | 15 | Glutaric | 9.00 | 60 | 2.5 | 4.56- 4.75* |
| 7 | 1-M-2-P | 3.1 | Succinic | 46.45 | 60 | 2.5 | 4.86 |
| 8 | 1-M-2-P | 3.1 | Succinic | 54.84 | 60 | 2.5 | 5.18 |
| 9 | dioxane | 15.9 | Glutaric | 3.06 | 60 | 2.5 | 4.36 |
| 10 | dioxane | 17.5 | Succinic | 3.77 | 60 | 2.5 | 4.6 |

| | | | | | | | |
|---|---|---|---|---|---|---|---|
| 1-M-2-P = 1-methoxy-2-propanol | | | | | | | |

### Example 11

86.5 g PEI polymer prepared according to Example 1 and 865 ml m are methoxy-2-propanol placed in a 2 Liter round bottom flask equipped with an agitator and reflux condenser and stirred for 30 min. 23.7 g maleic anhydride are added and the mixture stirred for 2.5 hr. at 60 °C. After allowing the mixture to cool, the mixture is transferred to a filter flask, drained, washed once with methoxy-2-propanol, three times with water, three times with methanol. Elemental analysis indicates that the final product contains: %C 55.6, %N 4.9. %H 7.1, %S 0.

### Example 12

86.5 g of a resin maleated as in Example 11 is heated in 900 ml of 0.01 N sodium hydroxide in the presence of 190 g of sodium meta-bisulfite for 6 hr at 80 +/-2 °C. After allowing the reaction mixture to cool, the mixture is transferred to a filter flask, drained, washed once with methoxy-2-propanol, three times with water, three times with methanol and stored in storage buffer for further use. Elemental analysis indicates that the final product contains: %C 49.8, %N 4.6. %H 6.8, %S 2.1.

### Example 13

The reaction was conducted as in Example 12 except the reaction is run for 20 hr at 80 +/-2 °C. Elemental analysis indicates that the final product contains: %C 50.2, %N 4.5, %H 6.8, %S 2.1.

Comparison of the sulfur content of samples obtained after 8 hrs of reaction (3.8% S, Example 12) and 20 hrs. of reaction (3.40% S, example 13) indicates that the reaction is complete after 8 hrs.

### Example 14

30 g PEI functionalized prepared according to Example 1 are washed twice with 120 ml 100% ethanol and twice with 120ml toluene to remove residual moisture. The material was then slurred in 300 ml of toluene, 9.29 g butyryl chloride (1 eq, based on nitrogen content of the resin) and 9.24 g of triethylamine were added and the reaction is allowed to proceed for 2 hr at 25 +/-2 °C. The resin was then transferred to a filter flask and washed with 300 ml of the toluene, 300 ml of methanol, twice with 300 ml of DI water, three times with 300 ml methanol and twice with 300 ml of pH 5 storage buffer (10mM sodium acetate, pH 4.5). Elemental analysis indicates that the final product contains: 53.7% C, 7.5 % H and 5.6 % N.

### Example 15

The reaction was conducted as in Example 14, except solvent is 450 ml dioxane. Elemental analysis indicates that the final product contains: 54.7% C, 8.0 % H and 5.5 % N.

### Example 16

The reaction was conducted as in Example 14, except 13.3 g butyryl chloride and 13.2 g triethylamine were used. Elemental analysis indicates that the final product contains: 53.6% C, 7.9 % H and 4.5 % N.

### Example 17

The reaction was conducted as in Example 14, except 19 g butyryl chloride and 18 g triethylamine were used. Elemental analysis indicates that the final product contains: 55.4% C, 7.8 % H and 4.2 % N.

### Example 18

The reaction was conducted as in Example 14, except 25.3 g butyryl chloride and 24 g triethylamine were used. Elemental analysis indicates that the final product contains: 55.0% C, 7.9 % H and 4.5 % N.

Some of the results obtained using toluene as the solvent are summarized in Table 3

**Table 3. Reaction of PEI coated resin with butyryl chloride in toluene (2 hr @ RT).**

| Example | Eq. Functionalization reagents/ eq. N | C/N | %N |
|---|---|---|---|
| 14 | 1 | 9.59 | 5.6 |
| 16 | 1.4 | 11.86 | 4.5 |
| 17 | 2 | 13.12 | 4.2 |
| 18 | 2.7 | 12.26 | 4.5 |

As the results in Table 3 indicate, increasing amounts of reagent result in an increased level of functionalization (as indicated by the corresponding decrease in nitrogen content of the product).

### Example 19

60 g PEI functionalized polymer prepared as in Example 1 and swollen in 200 ml of 1-methoxy-2-propanol, 600 ml 1-methoxy-2-propanol, 56.3 g butyric anhydride (1.5 molar excess) and 36 g triethylamine are reacted for 3 hr at 60 +/- 2 °C. The reaction mixture is transferred to a filter flask, drained, washed once with 500 ml 1-methoxy-2-propanopl, once with 500 ml methanol, twice with 500 ml DI water and twice with 500 ml storage buffer. Elemental analysis indicates that the final product contains: 54.6 % C, 7.9 %H and 4.8 % N.

### Example 20

60 g PEI functionalized polymer prepared as in Example 1 and swollen in 200 ml of 1-methoxy-2-propanol, 600 ml 1-methoxy-2-propanol, 6.2 g butyric anhydride and 4.0 g triethylamine are reacted for 3 hr at 60 +/- 2 °C. At that point, 24.2 g acetic anhydride were added and the reaction allowed to proceed at the same temperature for an additional 3 hr. The reaction mixture is transferred to a filter flask, drained, washed once with 500 ml 1-methoxy-2-propanopl, once with 500 ml methanol, twice with 500 ml DI water and twice with 500 ml storage buffer. Elemental analysis indicates that the final product contains: 53.7 % C, 7.7 %H and 4.7 % N.

### Example 21

The reaction was conducted as in Example 19 except 6.26 g butyric anhydride and 4.0 g triethylamine are used. Elemental analysis indicates that the final product contains: 53.7 % C, 7.9 %H and 4.8 % N.

### Example 22

The reaction was conducted as in Example 19 except 1.52 g butyric anhydride and 1.0 g triethylamine are used. Elemental analysis of the final product is 54.6 % C, 8.3 %H and 5.0 % N.

Some of the results are summarized in Table 4.

**Table 4. Reaction of PEI coated resin with butyric anhydride in 1-methoxy-2-propanol (3 hr. @ 60 °C)**

| Example | Eq. Functionalization reagents/ eq. N | C/N | %N |
|---|---|---|---|
| 20 | 1.5 | 11.4 | 4.7 |
| 21 | 0.16 | 11.2 | 4.8 |
| 22 | 0.04 | 10.9 | 5.0 |

Comparison of the results in Tables 3 and 4 shows that levels of substitutions similar to those obtained with butyryl chloride can be achieved with butyric anhydride using considerably lower stoichiometric amounts of reagent.

### Example 23

The reaction was conducted as in Example 20 except 1.52 g butyric anhydride and 1.0 g. triethylamine and 10.7 g acetic anhydride are used. Elemental analysis indicates that the final product contains: 55.4 % C, 7.9 %H and 4.9 % N.

### Example 24

60 g PEI functionalized polymer prepared as in Example 1 swollen in 20 ml of 1-methoxy-2-propanol, 500 ml 1-methoxy-2-propanol and 14.07 g acetic anhydride are reacted for 6 hr. at 60 +/- 2 °C. The reaction mixture is transferred to a filter flask, drained, washed once with 500 ml 1-methoxy-2-propanol, twice with 500 ml 0.1N NaOH, twice with 500 ml DI water and twice with 500 ml storage buffer. Elemental analysis indicates that the final product contains: 53.8 % C, 7.5 %H and 5.3 % N.

### Example 25

80 g PEI functionalized polymer prepared as in Example 1 is reacted for 8 hr at 80°C with a 59 g of a 60% solution of (3-chloro-2-hydroxypropyl) trimethyl ammonium chloride in 500 ml 0.5 N sodium hydroxide. The reaction mixture is transferred to a filter flask and washed twice with 0.1 N sodium hydroxide, twice with DI water and once with storage buffer. After washing and drying, the resin has the following elemental analysis: 54.2 %C, 8.4 %H, and 5.9% N.

### Example 26

The reaction was conducted as in Example 25 except the sodium hydroxide solution is 0.05N. After washing and drying, the resin has the following elemental analysis: 51.9 %C, 7.6 %H, and 5.3% N.

### Example 27

The reaction was conducted as in Example 25 except the reaction is continued for 16 hr. After washing and drying, the resin has the following elemental analysis: 53.4 %C, 8.4 %H, and 5.7% N.

### Example 28

The reaction was conducted as in Example 26 except the reaction is continued for 16 hr. After washing and drying, the resin has the following elemental analysis: 52.8 %C, 8.1 %H, and 5.7% N.

### Example 29

The reaction was conducted as in Example 25 except the ratio of (3-chloro-2-hydroxypropyl) trimethyl ammonium chloride 60% solution to resin is 0.4 instead of 0.73. After washing and drying, the resin has the following elemental analysis: 53.72 %C, 7.32 %H, and 6.04% N.

### Example 30

The reaction was conducted as in Example 25 except the ratio of (3-chloro-2-hydroxypropyl) trimethyl ammonium chloride 60% solution to resin is 1.2 instead of 0.73. After washing and drying, the resin has the following elemental analysis: 53.72%C, 7.32 %H, and 6.04% N.

**Table 5. Reaction of PEI coated resin with (3-chloro-2-hydroxypropyl) trimethylammonium chloride**

| Example | g funtionalization reagent/g resin | NaOH conc. | Reaction Time | %N |
|---|---|---|---|---|
| 25 | 0.74 | 0.5 N | 8 hr. | 5.9 |
| 26 | 0.74 | 0.05 N | 8 hr. | 5.3 |
| 27 | 0.74 | 0.5 N | 16 hr. | 5.7 |
| 28 | 0.74 | 0.05 N | 16 hr. | 5.7 |
| 29 | 0.4 | 0.5 N | 8 hr. | 6.04 |
| 30 | 1.2 | 0.5 N | 8 hr. | 6.04 |

### Example 31 (preparative)

160 g of PEI functionalized polymer prepared according to Examples 1 is reacted for 5 hours@ 40 °C with 111.8 g of formaldehyde, 400ml of acetonitrile and 25.9 g of sodium cyanoborohydride. After reaction, the polymer was washed with 480 ml of acetonitrile, 480 ml of DI water and 3 times 480 ml of methanol and stored in storage buffer for further use. (Elemental analysis %C = 55.2, %N = 5.8)

### Example 32

25 mg of polymer prepared according to Example 31 and 200 ml of acetonitrile wax charged to an autoclave (Parr series 4500 bench mounted pressure reactor with Parr 4840 temperature controller), While stirring the polymer at 120 RPM, methyl chloride gas was charged and pressurized to 60 PSI. The reaction was continued for 5 hours at 80 C. After reaction, the product was washed with 60ml actonitrile, 120 ml 0.1N sodium hydroxide, 120 ml DI water, 240 ml of storage buffer and stored In storage buffer.

### Example 33

71 g of PEI modified polymer prepared according to Example 1 was mixed with 500ml of 0.5N sodium hydroxide and reacted with 49 g of 3-(dimethylamino) propyl chloride while stirring at 50-100 RPM at 80°C for 8 hours. After redaction, the product was washed with twice with 500 ml sodium hydroxido, twice with 500 ml DI water, twice with 500ml of storage buffer and stored in the buffer for further use. Elemental analysis - %C: 55.4, %N: 6.2

### Example 34

### Chromatographic separation of proteins (illustrative)

A chromatographic media prepared as in Example 1 and PEI silica media from JT Baker (Product number 7264, lot N16084) packed in a 4.6 x 100 mm chromatographic column. 200 micro liters of a solution of 1 mg/ml lysozyme, 2 mg/ml rabbit immunoglobulin G, 2 mg/ml bovine serum albumin and 2 mg/ml each of beta-lactolobulin A and B in a 20 mM sodium acetate buffer at pH 6.2 is injected in the column and eluted using a flow rate of 1 ml/min and a 30 min gradient from 100 % of 20 mM sodium acetate buffer at pH 6.2 to 100% 1.0M sodium acetate buffer at pH 6.2. Elution of the proteins is recorded by a UV detector at 280 nm (Figure 1).

### Example 35

### Chromatographic separation of proteins

A chromatographic media prepared as in Example 9 is packed in a 4.6 x 100 mm chromatographic column. 200 micro liters of a solution of 4 mg/ml BSA, 2 mg/ml Rabbit IgG, 2mg/ml of cytochrome-c and 2mg/ml solution of lysozyme in 20 mM sodium acetate buffer at pH 6.2 is injected in the column and eluted using a flow rate of 1 ml/min and a 40 min gradient from 100 % of 20 mM sodium acetate buffer at pH 6.2 to 100% of the same eluent but containing 1 mole per liter of sodium acetate, pH 6.2. Elution of the proteins by the media of this Example and for a comparative silica based mixed mode media (JT Baker Product no. 7269) is recorded by a UV detector at 280 nm (Figure 2).

### Example 36

### Comparison of capacity

The capacity of chromatographic media prepared according to Example 9 and silica based products were measured and compared. A column (4.6 mm x 50mm) was packed with the media. IgG solution (1mg/ml) in 20mM sodium acetate at pH 5.6, 6.2 and 6.9 was applied to the column at varying linear velocity( cm/hr). The breakthrough was determined by monitoring UV at 280nm. The breakthrough capacity of the media in the column was determined at 10% breakthrough (Table 7).

**Table 7: Breakthrough Capacity of Mixed mode media and silica media**

| Samples | Linear Velocity, cm/hr | pH | Breakthrough Capacity, mg/ml |
|---|---|---|---|
| Example 9 | 361 | 6.2 | 11.9 |
| Example 9 | 722 | 6.2 | 10.7 |
| Mixed mode Silica Media-7269 | 361 | 6.2 | 5.3 |
| Mixed mode Silica Media-7269 | 722 | 6.2 | 5.9 |
| Example 9 | 361 | 5.6 | 47 |
| Example 9 | 722 | 5.6 | 32 |
| Mixed mode Silica Media-7269 | 361 | 5.6 | 15.5 |
| Mixed mode Silica Media-7269 | 722 | 5.6 | 13.1 |
| Example 9 | 361 | 6.9 | 7.1 |
| Mixed mode Silica Media-7269 | 361 | 6.9 | 3.5 |

### [Example 37

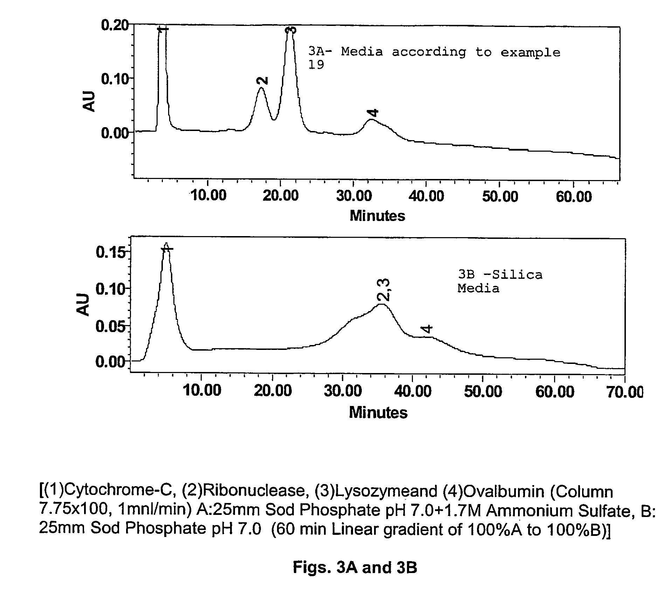

Chromatographic media prepared according to Example 19 and silica media (J T Baker product no. 7285) were packed in 7.75 mm x 100mm column. 200ul solution of 2mg/ml solution of Cytochrome C, ribonuclease, Lysozyme and Ovlbumin in 25mM sodium phosphate pH 7.0 +1.7M ammonium sulfate was injected. The protein was eluted from the column by 60 minute linear gradient of 100% 25mm sodium phosphate+1.7M ammonium sulfate to 100% of 25 mM sodium phosphate pH 7.0. The elution profile was monitored by UV @ 280 nm (Figure 3A and 3B).

### Example 38

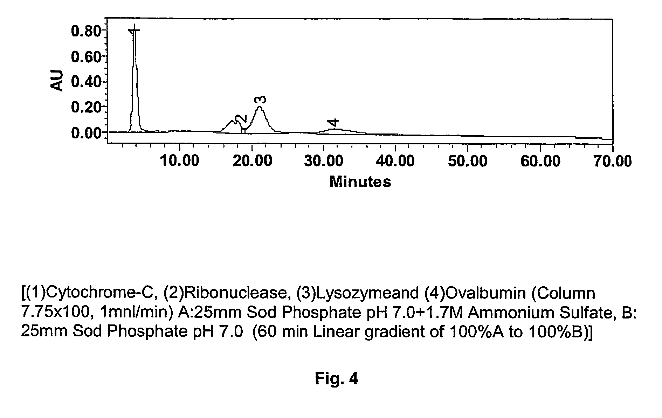

Chromatographic media prepared according to Example 20 was packed in 7.75 mm x 100 mm column. 200ul solution of 2mg/ml solution of Cytochrome C, ribonuclease, Lysozyme and Ovlbumin in 25mM sodium phosphate pH 7.0 +1.7M ammonium sulfate was injected. The protein was eluted from the column by 60 minute linear gradient of 100% 25mm sodium phosphate+1.7M ammonium sulfate to 100% of 25 mM sodium phosphate pH 7.0. The elution profile was monitored by UV @ 280 nm (Figure 4).

### Example 39

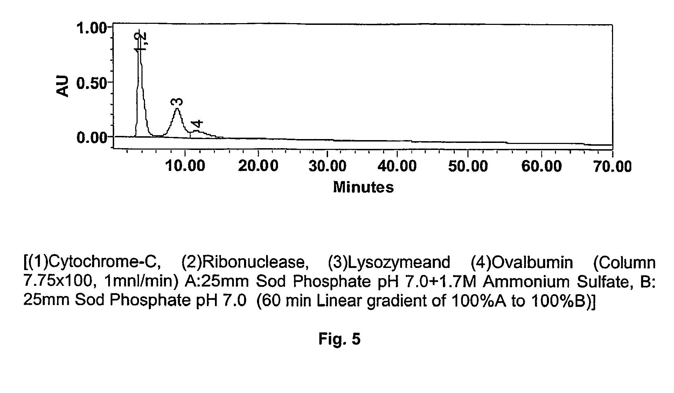

Chromatographic media prepared according to Example 22 was packed in 7.75 mm x 100mm column. 200ul solution of 2mg/ml solution of Cytochrome C, ribonuclease, Lysozyme and Ovlbumin in 25mM sodium phosphate pH 7.0 +1.7M ammonium sulfate was injected. The protein was eluted from the column by 60 minute linear gradient of 100% 25mm sodium phosphate+1.7M ammonium sulfate to 100% of 25 mM sodium phosphate pH 7.0. The elution profile was monitored by UV @ 280 nm (Figure 5).

### Example 40

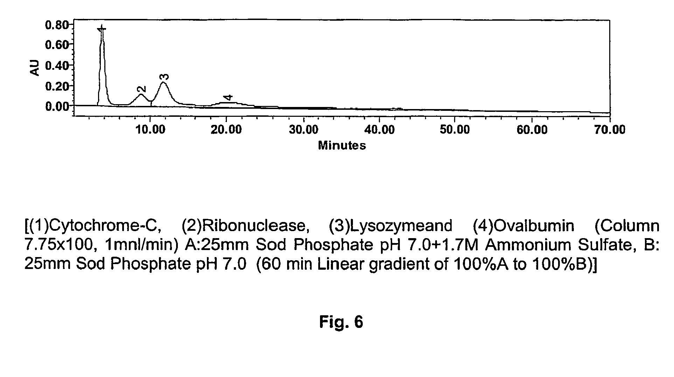

Chromatographic media prepared according to Example 23 was packed in 7.75 mm x 100mm column. 200ul solution of 2mg/ml solution of Cytochrome C, ribonuclease, Lysozyme and Ovalbumin in 25mM sodium phosphate pH 7.0 +1.7M ammonium sulfate was injected. The protein was eluted from the column by 60 minute linear gradient of 100% 25mm sodium phosphate+1.7M ammonium sulfate to 100% of 25 mM sodium phosphate pH 7.0. The elution profile was monitored by UV @ 280 nm (Figure 6).

### Example 41

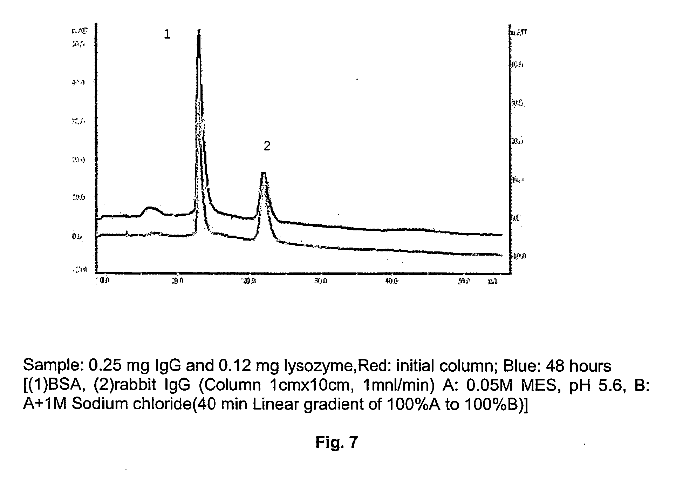

The polymer media prepared according to Example 7 was packed in 1.0cm x10cm column. First the column was equilibrated by passing 10 column volume of buffer A (0.05M MES, pH 5.6). After equilibration 0.5ml of protein solution containing rabbit globulin (0.5mg/ml) and lysozyme (0.25mg/ml) was injected. The proteins were eluted from the column by running 40 minute linear gradient of 100% buffer A to 100% buffer B (1 M Sodium chloride in buffer A). After the first protein separation run, the column was washed by circulating 500ml of 10mM phosphoric acid at 1ml/min for 48 hours. After washing the protein separation was carried out again to determine the acid stability of the column (Figure 7). This data indicates excellent stability of the polymeric mixed mode media under acidic conditions since there is no change in the retention time of IgG aand lysozyme before and after washing the column with phosphoric acid for up to 48 hours.

### Example 42

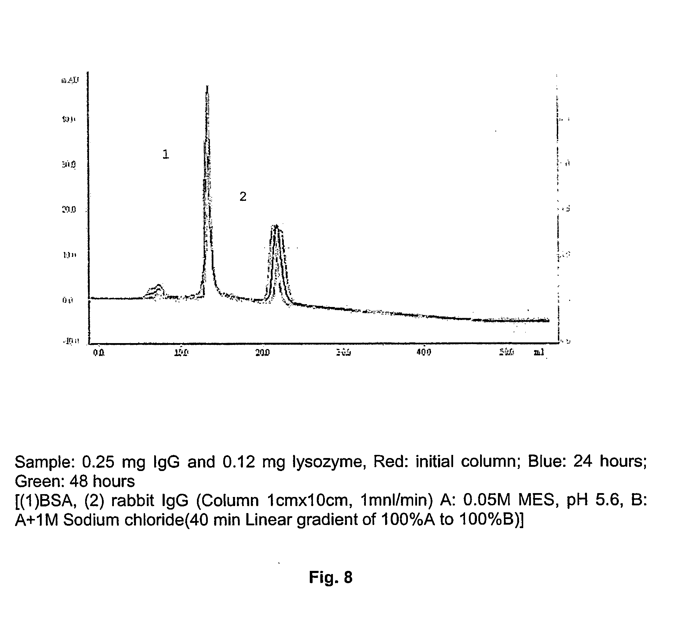

The polymer media prepared according to Example 7 was packed in 1.0cm x10cm column. First the column was equilibrated by passing 10 column volume of buffer A (0.05M MES, pH 5.6). After equilibration 0.5ml of protein solution containing rabbit globulin (0.5mg/ml) and lysozyme (0.25mg/ml) was injected. The proteins were eluted from the column by running 40 minute linear gradient of 100% buffer A to 100% buffer B (1M Sodium chloride in buffer A). After the first protein separation run, the column was washed by circulating 500ml of 0.1 M sodium hydroxide at 1ml/min for 24 and 48 hours. After washing the protein separation was carried out again to determine the base stability of the column (Figure 8). This data indicates excellent stability of the polymeric mixed mode media under basic conditions a there is no change in the retention time of IgG and lysozyme before and after washing the column with sodium hydroxide for up to 48 hours.

### Example 43

The capacity of chromatographic media prepared according to Example 1, 25, 26, 27, 28, 29, 30 and 32 were measured and compared. A column (4.6 mm x 50mm) was packed with the media. BSA solution (1mg/ml) in 20mM CAPS (3-[cyclohexylamino]-1-1 propane sulfonic acid) pH 11 was applied to the column at a flow rate of 1ml/min. The breakthrough was determined by monitoring UV at 280nm. The breakthrough capacity of the media in the column was determined at 10% breakthrough. After loading BSA on the columns, the columns were washed with CAPS buffer and then adsorbed BSA was eluted using 1M sodium chloride to calculate saturation capacity (Table 8).

**Table 8 Comparison of Capacity**

| Samples | Linear Velocity, cm/hr | PH | Breakthrough Capacity, mg/ml | Saturation Capacity, mg/ml |
|---|---|---|---|---|
| Example 1 preparative | 361 | 11 | 0.8 | 1 |
| Example 25 | 361 | 11 | 22 | 34 |
| Example 26 | 361 | 11 | 16 | 22 |
| Example 27 | 361 | 11 | 21 | 35 |
| Example 28 | 361 | 11 | 20 | 28 |
| Example 29 | 361 | 11 | 0.4 | 0.4 |
| Example 30 | 361 | 11 | 25 | 33 |
| Example 32 | 361 | 11 | 25 | 39 |

The invention has been described herein with reference to the specific embodiments thereof. It will be appreciated that changes, modification and variations can be made without departing from the scope of the claims. Accordingly, it is intended to embrace all such changes, modification and variations that fall with the scope of the appended claims.

## Claims

1. Chromatographic media comprising polymeric resin particles derivatized with polyethyleneimine on the surface of the polymer and functionalized by reaction of a functionalization reagent with terminal amino groups of the polyethyleneimine on the surface of the polymeric resin, wherein the functionalization reagent is selected from the group consisting of: acid anhydrides, sulfonation agents, alkyl chlorides, alkyl anhydrides, and alkyl chlorides containing quaternary ammonium functionality, and mixtures thereof.

2. Chromatographic media according to claim 1 wherein the functionalization reagent is selected from the group consisting of cyclic carboxylic anhydrides, unsaturated carboxylic anhydrides, bisulfites, alkyl chlorides, alkyl anhydrides, alkyl chlorides containing quaternary ammonium functionality and mixtures thereof.

3. Chromatographic media according to claim 2 wherein the functionalization reagent is selected from the group consisting of: glutaric anhydride, succinic anhydrides, maleic anhydride, sodium meta-bisulfite, burtryl chloride, acetic anhydride, butyric anhydride, (3-chloro-2-hydroxypropyl)trimethylammonium chloride, and mixtures thereof.

4. Chromatographic media according to any preceding claim In which the media Is mixed mode.

5. Chromatographic media according to claim 4 wherein the mixed mode media is selected from the group consisting of media with mixed primary, secondary and tertiary amine exchange sites, media with both weak anion and weak cation exchange sites, media with weak anion, weak cation and strong cation exchange sites, media with weak anion and hydrophobic (reverse phase) exchange sites, and media with weak anion and strong anion exchange sites.

6. A column for chromatography which is packed with chromatographic media according to any preceding claim.

7. A process for separation of components of a solution comprising passing the solution through a chromatography column of claim 6 and eluting components of the solution.

8. A process according to claim 7 wherein the solution is a solution containing proteins.

## Patentansprüche

1. Chromatographisches Medium aufweisend Polymerharzpartikel, die mit Polyethylenimin auf der Oberfläche des Polymers derivatisiert sind und durch eine Reaktion mit einem Funktionalisierungsmittel mit Amino-Endgruppen des Polyethylenimin auf der Oberfläche des Polymerharzes funktionalisiert sind, wobei das Funktionalisierungsmittel aus der Gruppe ausgewählt wurde, die aus sauren Anhydriden, Sulfonierungsmitteln, Alkylchloriden, Alkylanhydriden und Alkylchloriden mit quartiärer Ammonium-Funktionalität und Gemischen daraus besteht.

2. Chromatographisches Medium gemäß Anspruch 1 bei dem das Funktionalisierungsmittel aus der Gruppe ausgewählt wurde, die aus zyklischen carboxylischen Anhydriden, ungesättigten carboxylischen Anhydriden, Sulfiten, Alkylchloriden, Alkylanhydriden, Alkylchloriden mit quartiärer Ammonium-Funktionalität und Gemischen daraus besteht.

3. Chromatographisches Medium gemäß Anspruch 2 bei dem das Funktionalisierungsmittel aus der Gruppe ausgewählt wurde, die aus Glutar-Anhydriden, Bernsteinanhydriden, Maleinsäureanhydrid, Sodiummetha-Bisulfid, Butrylchlorid, Essigsäureanydrid, Buttersäureanhydrid, (3-Chlor-2-hydroxyprophyl)trimethylammoniumchlorid und Gemischen draus besteht.

4. Chromatographisches Medium gemäß einem der vorangegangenen Ansprüchen bei dem das Medium eine gemischte Form darstellt.

5. Chromatographisches Medium gemäß Anspruch 5, bei dem das in gemischter Form vorliegende Medium aus der Gruppe ausgewählt wurde bestehend aus: Mitteln gemischt mit primären, sekundären und tertiären Amino-Austauschplätzen, Mitteln mit schwachen Anionen- und schwachen Kationen-Austauschplätzen, Mitteln mit schwachen Anionen, schwachen und starken Kationen-Austauschplätzen, Mitteln mit schwachen Anionen und hydrophobischen (Umkehrphase) Austauschplätzen, und Mitteln mit schwachen und starken Anionen-Austauschseiten besteht.

6. Säule für Chromatographie, welche mit chromatographischen Medien gemäß einem der vorangegangenen Ansprüche ausgestattet ist.

7. Verfahren zur Trennung von Komponenten einer Lösung aufweisend das hindurch treten der Lösung durch eine Chromatographische Säule gemäß Anspruch 6 und eluieren der Komponenten der Lösung.

8. Verfahren nach Anspruch 7, bei dem die Lösung eine Lösung ist, die Proteine enthält.

## Revendications

1. Support chromatographique comprenant des particules d'une résine polymère transformées en dérivé avec de la polyéthylène-imine sur la surface du polymère et fonctionnalisées par réaction d'un réactif de fonctionnalisation avec les groupes amino terminaux de la polyéthylène-imine sur la surface de la résine polymère, dans lequel le réactif de fonctionnalisation est choisi dans le groupe consistant en : des anhydrides d'acides, des agents de sulfonation, des chlorures d'alkyle, des anhydrides d'alkyle, et des chlorures d'alkyle contenant une fonctionnalité ammonium quaternaire, ainsi que leurs mélanges.

2. Support chromatographique suivant la revendication 1, dans lequel le réactif de fonctionnalisation est choisi dans le groupe consistant en des anhydrides carboxyliques cycliques, des anhydrides carboxyliques insaturés, des bisulfites, des chlorures d'alkyle, des anhydrides d'alkyle, des chlorures d'alkyle contenant une fonctionnalité ammonium quaternaire et leurs mélanges.

3. Support chromatographique suivant la revendication 2, dans lequel le réactif de fonctionnalisation est choisi dans le groupe consistant en : l'anhydride glutarique, des anhydrides succiniques, l'anhydride maléique, le méta-bisulfite de sodium, le chlorure de butyryle, l'anhydride acétique, l'anhydride butyrique, le chlorure de (3-chloro-2-hydroxypropyl)triméthylammonium et leurs mélanges.

4. Support chromatographique suivant l'une quelconque des revendications précédentes, dans lequel le support est un support à mode mixte.

5. Support chromatographique suivant la revendication 4, dans lequel le support à mode mixte est choisi dans le groupe consistant en des supports avec des sites d'échange du type amine primaire, du type amine secondaire et du type amine tertiaire, des supports avec à la fois des sites d'échange d'anions faibles et des sites d'échange de cations faibles, des supports avec des sites d'échange d'anions faibles, de cations faibles et de cations forts, des supports avec des sites d'échange d'anions faibles et des sites d'échange hydrophobes (à phase inversée) et des supports avec des sites d'échange d'anions faibles et d'anions forts.

6. Colonne pour chromatographie qui est garnie d'un support chromatographique suivant l'une quelconque des revendications précédentes.

7. Procédé pour la séparation de constituants d'une solution, comprenant le passage de la solution à travers une colonne chromatographique de la revendication 6 et l'élution des constituants de la solution.

8. Procédé suivant la revendication 7, dans lequel la solution est une solution contenant des protéines.
